# FASCICULE DE BREVET EUROPEEN

(11) **EP 3 078 351 B1**
(45) Date de publication et mention de la délivrance du brevet: **17.05.2017**
(21) Numéro de dépôt: 16162769.0
(22) Date de dépôt: 30.03.2016
(51) Int. Cl.: A61F 2/38

(54) **IMPLANT TIBIAL POUR PROTHÈSE DE GENOU**
TIBIALIMPLANTAT FÜR KNIEPROTHESE
TIBIAL IMPLANT FOR KNEE PROSTHESIS

(30) Priorité: 08.04.2015 FR 1553003
(43) Date de publication de la demande: 12.10.2016
(73) Titulaire: Bonnin, Michel, 69006 Lyon (FR); Victor, Jan, 8301 Knokke-Heist (BE)
(72) Inventeur: Bonnin, Michel, 69006 Lyon (FR)
(74) Mandataire: Verriest, Philippe

(56) Documents cités:
- US-A- 5 964 808
- US-A1- 2004 006 393
- US-A1- 2010 016 976
- US-A1- 2013 245 777

## Description

La présente invention concerne un implant tibial pour prothèse de genou. Le document US 5964808 A est considéré comme l'état de la technique le plus proche et définit le préambule de la revendication 1. Une prothèse totale de genou comporte, de façon connue, d'une part un implant tibial comprenant une embase tibiale destinée à être fixée sur l'extrémité proximale réséquée du tibia d'un patient, et un insert tibial destiné à prendre appui sur l'embase tibiale, et d'autre part un composant fémoral destiné à être fixé sur l'extrémité distale réséquée du fémur et à prendre appui sur le plateau tibial.

Afin de limiter les risques de descellement de l'implant tibial et/ou de luxation du composant fémoral, il est connue de conformer l'embase tibiale et l'insert tibial de telle sorte qu'au moins l'un ou chacun de ces composants présente un profil extérieur correspondant sensiblement au contour cortical de l'extrémité proximale réséquée du tibia du patient à traiter. Une telle configuration de l'embase tibiale et de l'insert tibial permet en outre de limiter les risques de conflits avec les tissus mous à proximité de l'implant tibial.

Dans un genou sain, le tendon du muscle poplité s'étend le long et au contact de la zone postéro-latérale convexe de l'extrémité proximale du tibia, puis le long du bord latéral du condyle latéral fémoral, et enfin s'insert en bas et en avant de l'épicondyle latéral du fémur, antérieurement par rapport à la zone d'insertion du ligament collatéral latéral

Par conséquent, même lorsque l'insert tibial présente un profil extérieur parfaitement adapté au contour cortical de l'extrémité proximale réséquée du tibia du patient à traiter, l'insert tibial est susceptible d'entrer en conflit avec le tendon du muscle poplité, et donc d'induire des douleurs au patient. L'embase tibiale est également susceptible d'entrer en conflit avec le tendon du muscle poplité, et donc induire des douleurs au patient, lorsque l'embase tibiale présente une épaisseur importante et un profil extérieur parfaitement adapté au contour cortical de l'extrémité proximale réséquée du tibia du patient à traiter.

Afin d'éviter un tel conflit avec le tendon du muscle poplité, il pourrait être envisagé de sous-dimensionner l'insert tibial et/ou l'embase tibiale. Cependant, un tel sous-dimensionnement pourrait nuire à la stabilité et/ou à la fixation de la prothèse de genou, et ainsi induire des risques de descellement et/ou d'instabilité de la prothèse de genou.

La présente invention vise à remédier à ces inconvénients.

Le problème technique à la base de l'invention consiste donc à fournir un implant tibial pour prothèse de genou qui permette de limiter les risques de conflit avec le tendon du poplité, tout en limitant les risques de descellement et de luxation de la prothèse de genou.

A cet effet, la présente invention concerne un implant tibial pour prothèse de genou, comprenant :
- une embase tibiale destinée à être fixée sur une extrémité proximale réséquée d'un tibia d'un patient, l'embase tibiale comprenant une partie d'appui médiale et une partie d'appui latérale,
- un insert tibial conformé pour être monté sur l'embase tibiale, l'insert tibial comprenant :
   - une partie d'insert médiale comportant une surface d'appui médiale concave destinée à coopérer avec un condyle médial d'un composant fémoral ou d'un fémur, la partie d'insert médiale comprenant un bord postéro-médial et un bord antéro-médial, et
   - une partie d'insert latérale comportant une surface d'appui latérale concave destinée à coopérer avec un condyle latéral du composant fémoral ou du fémur, la partie d'insert latérale comprenant un bord postéro-latéral et un bord antéro-latéral,
caractérisé en ce que la partie d'insert latérale présente, au niveau de son bord postéro-latéral, une échancrure d'insert destinée au passage du tendon du poplité, et/ou en ce que la partie d'appui latérale présente, au niveau de son bord postéro-latéral, une échancrure d'embase destinée au passage du tendon du poplité.

La présence d'une telle échancrure de passage sur l'insert tibial et/ou l'embase tibiale permet d'éviter tout risque de conflit avec le tendon du poplité, quel que soit le dimensionnement de l'insert tibial et de l'embase tibiale. Ces dispositions permettent ainsi de conformer l'insert tibial et l'embase tibiale de telle sorte qu'au moins l'un ou chacun de ces composants présente un profil extérieur correspondant sensiblement au contour cortical de l'extrémité proximale réséquée du tibia du patient à traiter, et donc de limiter également les risques de descellement et d'instabilité de la prothèse de genou.

L'implant tibial peut en outre présenter une ou plusieurs des caractéristiques suivantes, prises seules ou en combinaison.

Selon un mode de réalisation de l'invention, l'échancrure d'insert est conformée de telle sorte qu'en conditions d'utilisation, le bord postéro-latéral de la partie d'insert latérale s'étend, au niveau de ladite échancrure d'insert, en retrait du contour cortical de l'extrémité proximale réséquée du tibia.

Selon un mode de réalisation de l'invention, l'échancrure d'insert est localisée dans un secteur angulaire centré sur l'axe d'intersection entre le plan antéro-postérieur médian et le plan médio-latéral médian de l'insert tibial, ledit secteur angulaire comportant un premier côté orienté selon un angle compris entre 20 et 40°, de préférence entre 25 et 35°, et par exemple d'environ 30°, par rapport au plan antéro-postérieur médian de l'insert tibial, et un deuxième côté orienté selon un angle compris entre 60 et 80°, de préférence entre 70 et 80°, et par exemple d'environ 80°, par rapport au plan antéro-postérieur médian de l'insert tibial.

Selon un mode de réalisation de l'invention, l'échancrure d'insert s'étend du premier côté jusqu'au deuxième côté du secteur angulaire dans lequel est localisée l'échancrure d'insert.

Selon un mode de réalisation de l'invention, l'échancrure d'insert s'étend sur une longueur comprise entre 5 et 10 mm.

Selon un mode de réalisation de l'invention, l'échancrure d'insert s'étend sur toute la hauteur de l'insert tibial.

Selon un mode de réalisation de l'invention, le bord antéro-latéral de la partie d'insert latérale et le bord antéro-médial de la partie d'insert médiale présentent des profils correspondant respectivement sensiblement aux contours corticaux antéro-latéral et antéro-médial de l'extrémité proximale réséquée du tibia.

Selon un mode de réalisation de l'invention, le bord postéro-médial de la partie d'insert médiale présente un profil correspondant sensiblement au contour cortical postéro-médial de l'extrémité proximale réséquée du tibia.

Selon un mode de réalisation de l'invention, le bord postéro-latéral de la partie d'insert latérale comporte une première portion de bord délimitant l'échancrure d'insert et au moins une deuxième portion de bord, la deuxième portion de bord présentant un profil correspondant sensiblement à une portion respective du contour cortical postéro-latéral de l'extrémité proximale réséquée du tibia.

Selon un mode de réalisation de l'invention, le bord postéro-latéral de la partie d'insert latérale comporte une troisième portion de bord présentant un profil correspondant sensiblement à une portion respective du contour cortical postéro-latéral de l'extrémité proximale réséquée du tibia, les deuxième et troisième portions de bord s'étendant de part et d'autre de la première portion de bord.

Selon un mode de réalisation de l'invention, l'échancrure d'embase est conformée de telle sorte qu'en conditions d'utilisation, le bord postéro-latéral de la partie d'appui latérale s'étend, au niveau de ladite échancrure d'embase, en retrait du contour cortical de l'extrémité proximale réséquée du tibia.

Selon un mode de réalisation de l'invention, l'échancrure d'embase est localisée dans un secteur angulaire centré sur l'axe d'intersection entre le plan antéro-postérieur médian et le plan médio-latéral médian de l'embase tibiale, ledit secteur angulaire comportant un premier côté orienté selon un angle compris entre 20 et 40°, de préférence entre 25 et 35°, et par exemple d'environ 30°, par rapport au plan antéro-postérieur médian de l'embase tibiale, et un deuxième côté orienté selon un angle compris entre 60 et 80°, de préférence entre 70 et 80°, et par exemple d'environ 80°, par rapport au plan antéro-postérieur médian de l'embase tibiale.

Selon un mode de réalisation de l'invention, l'échancrure d'embase s'étend du premier côté jusqu'au deuxième côté du secteur angulaire dans lequel est localisée l'échancrure d'embase.

Selon un mode de réalisation de l'invention, l'échancrure d'embase s'étend sur une longueur comprise entre 5 et 10 mm.

Selon un mode de réalisation de l'invention, l'échancrure d'embase s'étend sur toute la hauteur de l'embase tibiale.

Selon un mode de réalisation de l'invention, la partie d'appui médiale comprend un bord postéro-médial et un bord antéro-médial.

Selon un mode de réalisation de l'invention, le bord antéro-latéral de la partie d'appui latérale et le bord antéro-médial de la partie d'appui médiale présentent des profils correspondant respectivement sensiblement aux contours corticaux antéro-latéral et antéro-médial de l'extrémité proximale réséquée du tibia.

Selon un mode de réalisation de l'invention, le bord postéro-médial de la partie d'appui médiale présente un profil correspondant sensiblement au contour cortical postéro-médial de l'extrémité proximale réséquée du tibia.

Selon un mode de réalisation de l'invention, le bord postéro-latéral de la partie d'appui latérale comporte une première portion de bord délimitant l'échancrure d'embase et au moins une deuxième portion de bord, la deuxième portion de bord présentant un profil correspondant sensiblement à une portion respective du contour cortical postéro-latéral de l'extrémité proximale réséquée du tibia.

Selon un mode de réalisation de l'invention, le bord postéro-latéral de la partie d'appui latérale comporte une troisième portion de bord présentant un profil correspondant sensiblement à une portion respective du contour cortical postéro-latéral de l'extrémité proximale réséquée du tibia, les deuxième et troisième portions de bord du bord postéro-latéral de la partie d'appui latérale s'étendant de part et d'autre de la première portion de bord respective.

Selon un mode de réalisation de l'invention, l'embase tibiale présente un profil extérieur sensiblement identique au profil extérieur de l'insert tibial.

Selon un mode de réalisation de l'invention, l'insert tibial est conformé pour être monté fixe sur l'embase tibiale.

Selon un autre mode de réalisation de l'invention, l'insert tibial est conformé pour être monté mobile en rotation sur l'embase tibiale.

Selon un mode de réalisation de l'invention, l'embase tibiale comprend au moins un élément de fixation, tel qu'une quille ou tige de fixation, destiné à être fixé sur l'extrémité proximale réséquée du tibia.

Selon un mode de réalisation de l'invention, l'embase tibiale est métallique.

Selon un mode de réalisation de l'invention, l'insert tibial est réalisé en polyéthylène, et par exemple en polyéthylène à très haut poids moléculaire.

De toute façon l'invention sera bien comprise à l'aide de la description qui suit en référence au dessin schématique annexé représentant, à titre d'exemples non limitatifs, plusieurs formes d'exécution de cet implant tibial.
Figure 1 est une vue en perspective d'un implant tibial selon un premier mode de réalisation de l'invention.
Figure 2 est une vue de dessus d'une embase tibiale de l'implant tibial de la figure 1.
Figure 3 est une vue de dessus d'un insert tibial de l'implant tibial de la figure 1.
Figure 4 est une vue schématique de dessus d'un insert tibial d'un implant tibial selon un deuxième mode de réalisation de l'invention.
Figure 5 est une vue schématique de dessus d'un insert tibial d'un implant tibial selon un troisième mode de réalisation de l'invention.
Figure 6 est une vue schématique de dessus d'un insert tibial d'un implant tibial selon un quatrième mode de réalisation de l'invention.
Figures 7 et 8 sont des vues de dessus d'un implant tibial selon un cinquième mode de réalisation de l'invention, dans deux positions de fonctionnement différentes.

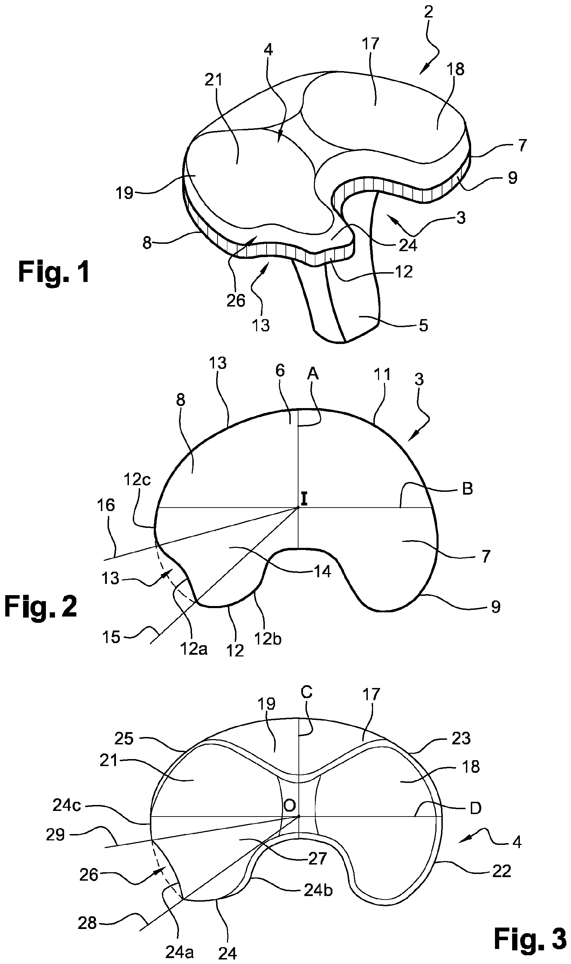

Les figures 1 à 3 représentent un implant tibial 2 pour prothèse de genou selon premier mode de réalisation de l'invention. L'implant tibial 2 comprend une embase tibiale 3 destinée à être fixée sur une extrémité proximale réséquée d'un tibia d'un patient, et un insert tibial 4 conformé pour être monté sur l'embase tibiale 3. L'embase tibiale peut par exemple être métallique, et l'insert tibial peut être réalisé en polyéthylène, et par exemple en polyéthylène à très haut poids moléculaire.

Comme montré sur la figure 1, l'embase tibiale 3 comprend au moins un élément de fixation 5, tel qu'une quille ou tige de fixation, destiné à être fixé sur l'extrémité proximale réséquée du tibia. L'embase tibiale 3 comprend en outre une partie d'appui 6 destinée à prendre appui sur l'extrémité proximale réséquée du tibia. La partie d'appui 6 comprend une partie d'appui médiale 7 et une partie d'appui latérale 8.

La partie d'appui médiale 7 comprend un bord postéro-médial 9 s'étendant sur une portion postérieure de l'embase tibiale 3, et plus particulièrement depuis le plan antéro-postérieur médian A, également nommé plan sagittal médian, de l'embase tibiale 3 jusqu'au plan médio-latéral médian B, également nommé plan frontal médian, de l'embase tibiale 3. La partie d'appui médiale 7 comprend également un bord antéro-médial 11 s'étendant sur une portion antérieure de l'embase tibiale 3, et plus particulièrement depuis le plan médio-latéral médian B jusqu'au plan antéro-postérieur médian A.

La partie d'appui latérale 8 comprend un bord postéro-latéral 12 s'étendant sur une portion postérieure de l'embase tibiale 3, et plus particulièrement depuis le plan antéro-postérieur médian A jusqu'au plan médio-latéral médian B. La partie d'appui latérale 8 comprend également un bord antéro-latéral 13 s'étendant sur une portion antérieure de l'embase tibiale 3, et plus particulièrement depuis le plan médio-latéral médian B jusqu'au plan antéro-postérieur médian A.

Selon le premier mode de réalisation représenté sur les figures 1 à 3, la partie d'appui latérale 8 présente, au niveau de son bord postéro-latéral 12, une échancrure d'embase 13 destinée au passage du tendon du poplité. L'échancrure d'embase 13 s'étend sur toute la hauteur de l'embase tibiale 3, et est localisée dans un secteur angulaire 14 centré sur l'axe d'intersection I entre le plan antéro-postérieur médian A et le plan médio-latéral médian B. Le secteur angulaire 14 comporte un premier côté 15 orienté selon un angle compris avantageusement entre 20 et 40°, de préférence entre 25 et 35°, et par exemple d'environ 30°, par rapport au plan antéro-postérieur médian A, et un deuxième côté 16 orienté selon un angle compris avantageusement entre 60 et 80°, de préférence entre 70 et 80°, et par exemple d'environ 80°, par rapport au plan antéro-postérieur médian A. L'échancrure d'embase 13 s'étend avantageusement du premier côté 15 jusqu'au deuxième côté 16 du secteur angulaire 14.

L'échancrure d'embase 13 est conformée de telle sorte qu'en conditions d'utilisation, le bord postéro-latéral 12 de la partie d'appui latérale 8 s'étend, au niveau de l'échancrure d'embase 13, en retrait du contour cortical de l'extrémité proximale réséquée du tibia. La portion du contour cortical de l'extrémité proximale réséquée du tibia s'étendant au niveau de l'échancrure d'embase 13 est schématisée en pointillé sur la figure 2.

Selon le premier mode de réalisation représenté sur les figures 1 à 3, le bord antéro-latéral 13 de la partie d'appui latérale 8 et le bord antéro-médial 11 de la partie d'appui médiale 7 présentent des profils correspondant respectivement sensiblement aux contours corticaux antéro-latéral et antéro-médial de l'extrémité proximale réséquée du tibia. Le bord postéro-médial 9 de la partie d'appui médiale 7 présente également un profil correspondant sensiblement au contour cortical postéro-médial de l'extrémité proximale réséquée du tibia.

Selon le premier mode de réalisation représenté sur les figures 1 à 3, le bord postéro-latéral 12 de la partie d'appui latérale 8 comporte une première portion de bord 12a délimitant l'échancrure d'embase 13, une deuxième portion de bord 12b et une troisième portion de bord 12c, les deuxième et troisième portions de bord 12b, 12c s'étendant de part et d'autre de la première portion de bord 12a. Chacune des deuxième et troisième portions de bord 12b, 12c présente avantageusement un profil correspondant sensiblement à une portion respective du contour cortical postéro-latéral de l'extrémité proximale réséquée du tibia. Ainsi, l'embase tibiale 3 présente avantageusement, sauf au niveau de l'échancrure d'embase 13, un profil extérieur correspondant sensiblement au contour cortical de l'extrémité proximale réséquée du tibia.

Comme montré sur les figures 1 et 3, l'insert tibial 4 présente avantageusement un profil extérieur sensiblement identique au profil extérieur de l'embase tibiale 3. L'insert tibial 4 comprend une partie d'insert médiale 17 comportant une surface d'appui médiale concave 18 destinée à coopérer avec un condyle médial d'un composant fémoral ou d'un fémur, et une partie d'insert latérale 19 comportant une surface d'appui latérale concave 21 destinée à coopérer avec un condyle latéral du composant fémoral ou du fémur. Selon le mode de réalisation représenté sur les figures 1 à 3, l'insert tibial 4 est conformé pour être monté fixe sur l'embase tibiale 3. A cet effet, l'insert tibial 4 et l'embase tibiale 3 comportent des éléments de fixation complémentaires (non représentés sur les figures) bien connus de l'homme du métier.

La partie d'insert médiale 17 comprend un bord postéro-médial 22 s'étendant sur une portion postérieure de l'insert tibial 4, et plus particulièrement depuis un plan antéro-postérieur médian C, également nommé plan sagittal médian, de l'insert tibial 4 jusqu'à un plan médio-latéral médian D, également nommé plan frontal médian, de l'insert tibial 4. La partie d'insert médiale 17 comprend également un bord antéro-médial 23 s'étendant sur une portion antérieure de l'insert tibial 4, et plus particulièrement depuis le plan médio-latéral médian D jusqu'au plan antéro-postérieur médian C.

La partie d'insert latérale 19 comprend un bord postéro-latéral 24 s'étendant sur une portion postérieure de l'insert tibial 4, et plus particulièrement depuis le plan antéro-postérieur médian C jusqu'au plan médio-latéral médian D, et un bord antéro-latéral 25 s'étendant sur une portion antérieure de l'insert tibial 4, et plus particulièrement depuis le plan médio-latéral médian D jusqu'au plan antéro-postérieur médian C.

Selon le premier mode de réalisation représenté sur les figures 1 à 3, la partie d'insert latérale 19 présente, au niveau de son bord postéro-latéral 24, une échancrure d'insert 26 destinée au passage du tendon du poplité. L'échancrure d'insert 26 s'étend sur toute la hauteur de l'embase tibiale 3, et est localisée dans un secteur angulaire 27 centré sur l'axe d'intersection O entre le plan antéro-postérieur médian C et le plan médio-latéral médian D. Le secteur angulaire 27 comporte un premier côté 28 orienté selon un angle compris avantageusement entre 20 et 40°, de préférence entre 25 et 35°, et par exemple d'environ 30°, par rapport au plan antéro-postérieur médian C, et un deuxième côté 29 orienté selon un angle compris avantageusement entre 60 et 80°, de préférence entre 70 et 80°, et par exemple d'environ 80°, par rapport au plan antéro-postérieur médian C. L'échancrure d'insert 26 s'étend avantageusement du premier côté 28 jusqu'au deuxième côté 29 du secteur angulaire 27.

L'échancrure d'insert 26 est conformée de telle sorte qu'en conditions d'utilisation, le bord postéro-latéral 24 de la partie d'insert latérale 19 s'étend, au niveau de l'échancrure d'insert 26, en retrait du contour cortical de l'extrémité proximale réséquée du tibia. La portion du contour cortical de l'extrémité proximale réséquée du tibia s'étendant au niveau de l'échancrure d'insert 26 est schématisée en pointillé sur la figure 3.

Selon le premier mode de réalisation représenté sur les figures 1 à 3, le bord antéro-latéral 25 de la partie d'insert latérale 19 et le bord antéro-médial 23 de la partie d'insert médiale 17 présentent des profils correspondant respectivement sensiblement aux contours corticaux antéro-latéral et antéro-médial de l'extrémité proximale réséquée du tibia. Le bord postéro-médial 22 de la partie d'insert médiale 17 présente également un profil correspondant sensiblement au contour cortical postéro-médial de l'extrémité proximale réséquée du tibia.

Selon le premier mode de réalisation représenté sur les figures 1 à 3, le bord postéro-latéral 24 de la partie d'insert latérale 19 comporte une première portion de bord 24a délimitant l'échancrure d'insert 26, une deuxième portion de bord 24b et une troisième portion de bord 24c, les deuxième et troisième portions de bord 24b, 24c s'étendant de part et d'autre de la première portion de bord 24a. Chacune des deuxième et troisième portions de bord 24b, 24c présente avantageusement un profil correspondant sensiblement à une portion respective du contour cortical postéro-latéral de l'extrémité proximale réséquée du tibia. Ainsi, l'insert tibial 4 présente avantageusement, sauf au niveau de l'échancrure d'insert 26, un profil extérieur correspondant sensiblement au contour cortical de l'extrémité proximale réséquée du tibia.

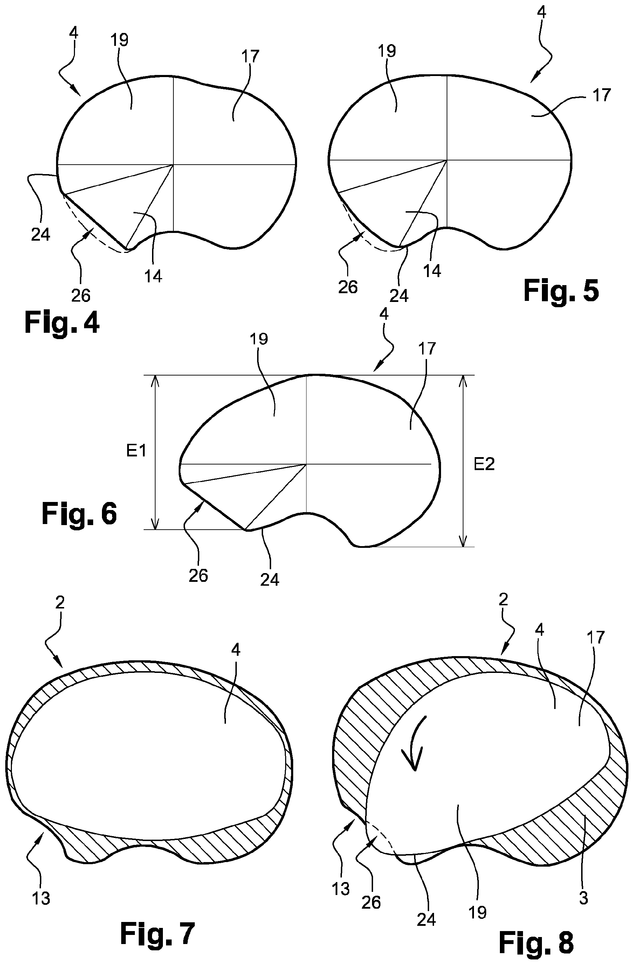

La figure 4 représente schématiquement un insert tibial 4 d'un implant tibial selon un deuxième mode de réalisation de l'invention qui diffère de celui représenté sur les figures 1 à 3 essentiellement en ce que l'échancrure d'insert 26 est formée par un méplat. Selon un tel mode de réalisation de l'invention, l'embase tibiale 3 pourrait présenter un profil extérieur sensiblement identique au profil extérieur de l'insert tibial 4 représenté sur la figure 4, et ainsi l'échancrure d'embase 13 pourrait être formée par un méplat.

La figure 5 représente schématiquement un insert tibial 4 d'un implant tibial selon un troisième mode de réalisation de l'invention qui diffère de celui représenté sur les figures 1 à 3 essentiellement en ce que l'échancrure d'insert 26 est formée par une variation du rayon de courbure du bord postéro-latéral 24 de la partie d'insert latérale 19, et plus particulièrement de la première portion de bord 12a. Selon un tel mode de réalisation de l'invention, l'embase tibiale 3 pourrait présenter un profil extérieur sensiblement identique au profil extérieur de l'insert tibial 4 représenté sur la figure 5, et ainsi l'échancrure d'embase 13 pourrait être formée par une variation du rayon de courbure du bord postéro-latéral 12 de la partie d'appui latérale 8.

La figure 6 représente schématiquement un insert tibial 4 d'un implant tibial selon un quatrième mode de réalisation de l'invention qui diffère de celui représenté sur les figures 1 à 3 essentiellement en ce que l'insert tibial 4 est asymétrique, c'est-à-dire que l'encombrement antéro-postérieur E1 de la partie d'insert latéral 19 est inférieur à l'encombrement antéro-postérieur E2 de la partie d'insert médial 17. Selon un tel mode de réalisation de l'invention, l'embase tibiale 3 pourrait présenter un profil extérieur sensiblement identique au profil extérieur de l'insert tibial 4 représenté sur la figure 6, et ainsi l'encombrement antéro-postérieur de la partie d'appui latéral 8 pourrait être inférieur à l'encombrement antéro-postérieur de la partie d'appui médial 7.

Les figures 7 et 8 représentent un implant tibial 2 selon un cinquième mode de réalisation de l'invention qui diffère de celui représenté sur les figures 1 à 3 essentiellement en ce que l'insert tibial 4 est conformé pour être monté mobile en rotation sur l'embase tibiale 3. A cet effet, l'insert tibial 4 et l'embase tibiale 3 comportent des moyens d'assemblage adaptés bien connus de l'homme du métier.

Selon tel mode de réalisation de l'invention, l'insert tibial 4 pourrait éventuellement être dépourvu d'échancrure d'insert 26. Néanmoins, dans le cas où l'insert tibial 4 est pourvu d'une échancrure d'insert 26 (représentée en pointillé sur la figure 8), alors celle-ci est conformée de telle sorte que lors d'une rotation externe maximale de l'insert tibial 4, le bord postéro-latéral 24 de la partie d'insert latérale 19 s'étend, au niveau de ladite échancrure d'insert 26, en retrait du contour cortical de l'extrémité proximale réséquée du tibia.

Comme il va de soi, l'invention ne se limite pas à la seule forme d'exécution de cet implant tibial, décrite ci-dessus à titre d'exemple, elle en embrasse au contraire toutes les variantes de réalisation.

## Revendications

1. Implant tibial (2) pour prothèse de genou, comprenant :
- une embase tibiale (3) destinée à être fixée sur une extrémité proximale réséquée d'un tibia d'un patient, l'embase tibiale (3) comprenant une partie d'appui médiale (7) et une partie d'appui latérale (8), la partie d'appui latérale (8) comprenant un bord postéro-latéral (12) et un bord antéro-latéral (13),
- un insert tibial (4) conformé pour être monté sur l'embase tibiale (3), l'insert tibial (4) comprenant :
- une partie d'insert médiale (17) comportant une surface d'appui médiale concave (18) destinée à coopérer avec un condyle médial d'un composant fémoral ou d'un fémur, la partie d'insert médiale (18) comprenant un bord postéro-médial (22) et un bord antéro-médial (23), et
- une partie d'insert latérale (19) comportant une surface d'appui latérale concave (21) destinée à coopérer avec un condyle latéral du composant fémoral ou du fémur, la partie d'insert latérale (19) comprenant un bord postéro-latéral (24) et un bord antéro-latéral (25),
**caractérisé en ce que** la partie d'insert latérale (19) présente, au niveau de son bord postéro-latéral (24), une échancrure d'insert (26) destinée au passage du tendon du poplité, et/ou **en ce que** la partie d'appui latérale (8) présente, au niveau de son bord postéro-latéral (12), une échancrure d'embase (13) destinée au passage du tendon du poplité.

2. Implant tibial (2) selon la revendication 1, dans lequel l'échancrure d'insert (26) est conformée de telle sorte qu'en conditions d'utilisation, le bord postéro-latéral (24) de la partie d'insert latérale (19) s'étend, au niveau de ladite échancrure d'insert (26), en retrait du contour cortical de l'extrémité proximale réséquée du tibia.

3. Implant tibial (2) selon la revendication 1 ou 2, dans lequel l'échancrure d'insert (26) est localisée dans un secteur angulaire (27) centré sur l'axe d'intersection (O) entre le plan antéro-postérieur médian (C) et le plan médio-latéral médian (D) de l'insert tibial (4), ledit secteur angulaire (27) comportant un premier côté (28) orienté selon un angle compris entre 20 et 40° par rapport au plan antéro-postérieur médian (C) de l'insert tibial (4), et un deuxième côté (29) orienté selon un angle compris entre 60 et 80° par rapport au plan antéro-postérieur médian (C) de l'insert tibial (4).

4. Implant tibial (2) selon l'une quelconque des revendications 1 à 3, dans lequel l'échancrure d'insert (26) s'étend sur toute la hauteur de l'insert tibial (4).

5. Implant tibial (2) selon l'une quelconque des revendications 1 à 4, dans lequel le bord antéro-latéral (25) de la partie d'insert latérale (19) et le bord antéro-médial (23) de la partie d'insert médiale (17) présentent des profils correspondant respectivement sensiblement aux contours corticaux antéro-latéral et antéro-médial de l'extrémité proximale réséquée du tibia.

6. Implant tibial (2) selon l'une quelconque des revendications 1 à 5, dans lequel le bord postéro-médial (22) de la partie d'insert médiale (17) présente un profil correspondant sensiblement au contour cortical postéro-médial de l'extrémité proximale réséquée du tibia.

7. Implant tibial (2) selon l'une quelconque des revendications 1 à 6, dans lequel le bord postéro-latéral (24) de la partie d'insert latérale (19) comporte une première portion de bord (24a) délimitant l'échancrure d'insert (26) et au moins une deuxième portion de bord (24b), la deuxième portion de bord (24b) présentant un profil correspondant sensiblement à une portion respective du contour cortical postéro-latéral de l'extrémité proximale réséquée du tibia.

8. Implant tibial (2) selon l'une quelconque des revendications 1 à 7, dans lequel l'échancrure d'embase (13) est conformée de telle sorte qu'en conditions d'utilisation, le bord postéro-latéral (12) de la partie d'appui latérale (8) s'étend, au niveau de ladite échancrure d'embase (13), en retrait du contour cortical de l'extrémité proximale réséquée du tibia.

9. Implant tibial (2) selon l'une quelconque des revendications 1 à 8, dans lequel l'échancrure d'embase (13) est localisée dans un secteur angulaire (14) centré sur l'axe d'intersection (I) entre le plan antéro-postérieur médian (A) et le plan médio-latéral médian (B) de l'embase tibiale (3), ledit secteur angulaire (14) comportant un premier côté (15) orienté selon un angle compris entre 20 et 40° par rapport au plan antéro-postérieur médian (A) de l'embase tibiale (3), et un deuxième côté (16) orienté selon un angle compris entre 60 et 80° par rapport au plan antéro-postérieur médian (A) de l'embase tibiale (3).

10. Implant tibial (2) selon l'une quelconque des revendications 1 à 9, dans lequel l'échancrure d'embase (13) s'étend sur toute la hauteur de l'embase tibiale (3).

11. Implant tibial (2) selon l'une quelconque des revendications 1 à 10, dans lequel l'embase tibiale (3) présente un profil extérieur sensiblement identique au profil extérieur de l'insert tibial (4).

12. Implant tibial (2) selon l'une quelconque des revendications 1 à 11, dans lequel l'insert tibial (4) est conformé pour être monté fixe sur l'embase tibiale (3).

## Patentansprüche

1. Schienbeinimplantat (2) für Knieprothese, das Folgendes umfasst:
- eine Schienbeingrundplatte (3), die dazu bestimmt ist, auf einem resezierten proximalen Ende eines Schienbeins eines Patienten befestigt zu sein, wobei die Schienbeingrundplatte (3) einen medialen Auflageteil (7) und einen lateralen Auflageteil (8) umfasst, wobei der laterale Auflageteil (8) einen posterolateralen Rand (12) und einen anterolateralen Rand (13) umfasst,
- einen Schienbeineinsatz (4), der ausgestaltet ist, um auf die Schienbeingrundplatte (3) montiert zu sein, wobei der Schienbeineinsatz (4) Folgendes umfasst:
- einen medialen Einsatzteil (17), der eine konkave mediale Auflageoberfläche (18) umfasst, die dazu bestimmt ist, mit einem medialen Kondylus eines femoralen Bauteils oder mit einem Oberschenkelknochen zusammenzuwirken, wobei der mediale Einsatzteil (18) einen posteromedialen (22) und einen anteromedialen Rand (23) umfasst, und
- einen lateralen Einsatzteil (19), der eine konkave laterale Auflageoberfläche (21) umfasst, die dazu bestimmt ist, mit einem lateralen Kondylus des femoralen Bauteils oder mit dem Oberschenkelknochen zusammenzuwirken, wobei der laterale Einsatzteil (19) einen posterolateralen (24) und einen anterolateralen (25) Rand umfasst,
**dadurch gekennzeichnet, dass** der laterale Einsatzteil (19) im Bereich seines posterolateralen Rands (24) einen Einsatzausschnitt (26) umfasst, der für das Durchgehen der Kniekehlensehne bestimmt ist, und/oder dass der laterale Auflageteil (8) im Bereich seines posterolateralen Rands (12) einen Grundplattenausschnitt (13) aufweist, der für das Durchgehen der Kniekehlensehne bestimmt ist.

2. Schienbeinimplantat (2) nach Anspruch 1, bei dem der Einsatzausschnitt (26) derart ausgestaltet ist, dass sich der posterolaterale Rand (24) des lateralen Einsatzteils (19) unter Gebrauchsbedingungen im Bereich des Einsatzausschnitts (26), von der kortikalen Kontur des resezierten proximalen Endes des Schienbeins zurück versetzt erstreckt.

3. Schienbeinimplantat (2) nach Anspruch 1 oder 2, wobei der Einsatzausschnitt (26) in einem Winkelsektor (27) liegt, der zwischen der Schnittachse (O) zwischen der medianen anteroposterioren Ebene (C) und der medianen mediolateralen Ebene (D) des Schienbeineinsatzes (4) zentriert ist, wobei der Winkelsektor (27) eine erste Seite (28) umfasst, die gemäß einem Winkel zwischen 20 und 40° in Bezug zu der medianen anteroposterioren Ebene (C) des Schienbeineinsatzes (4) ausgerichtet ist, und eine zweite Seite (29), die gemäß einem Winkel zwischen 60 und 80° in Bezug zu der medianen anteroposterioren Ebene (C) des Schienbeineinsatzes (4) ausgerichtet ist.

4. Schienbeinimplantat (2) nach einem der Ansprüche 1 bis 3, wobei sich der Einsatzausschnitt (26) auf der gesamten Höhe des Schienbeineinsatzes (4) erstreckt.

5. Schienbeinimplantat (2) nach einem der Ansprüche 1 bis 4, wobei der anterolaterale Rand (25) des lateralen Einsatzteils (19) und der anteromediale Rand (23) des medialen Einsatzteils (17) Profile aufweisen, die jeweils im Wesentlichen der anterolateralen und anteromedialen kortikalen Kontur des resezierten proximalen Endes des Schienbeins entsprechen.

6. Schienbeinimplantat (2) nach einem der Ansprüche 1 bis 5, wobei der posteromediale Rand (22) des medialen Einsatzteils (17) ein Profil aufweist, das im Wesentlichen der posteromedialen kortikalen Kontur des resezierten proximalen Endes des Schienbeins entspricht.

7. Schienbeinimplantat (2) nach einem der Ansprüche 1 bis 6, wobei der posterolaterale Rand (24) des lateralen Einsatzteils (19) einen ersten Randabschnitt (24a) umfasst, der den Einsatzausschnitt (26) abgrenzt, und mindestens einen zweiten Randabschnitt (24b), wobei der zweite Randabschnitt (24b) ein Profil aufweist, das im Wesentlichen einem jeweiligen Abschnitt der posterolateralen kortikalen Kontur des resezierten proximalen Endes des Schienbeins entspricht.

8. Schienbeinimplantat (2) nach einem der Ansprüche 1 bis 7, wobei der Grundplattenausschnitt (13) derart ausgestaltet ist, dass sich der posterolaterale Rand (12) des lateralen Auflageteils (8) unter Gebrauchsbedingungen im Bereich des Grundplattenausschnitts (13) von der kortikalen Kontur des resezierten proximalen Endes des Schienbeins zurück versetzt erstreckt.

9. Schienbeinimplantat (2) nach einem der Ansprüche 1 bis 8, wobei sich der Grundplattenausschnitt (13) in einem Winkelsektor (14) befindet, der auf der Schnittstellenachse (I) zwischen der medianen anteroposterioren Ebene (A) und der medianen mediolateralen Ebene (B) der Schienbeingrundplatte (3) zentriert ist, wobei der Winkelsektor (14) eine erste Seite (15) umfasst, die gemäß einem Winkel zwischen 20 und 40° in Bezug zu der medianen anteroposterioren Ebene (A) der Schienbeingrundplatte (3) ausgerichtet ist, und eine zweite Seite (16), die gemäß einem Winkel zwischen 60 und 80° in Bezug zu der medianen anteroposterioren Ebene (A) der Schienbeingrundplatte (3) ausgerichtet ist.

10. Schienbeinimplantat (2) nach einem der Ansprüche 1 bis 9, wobei sich der Grundplattenausschnitt (13) auf der gesamten Höhe der Schienbeingrundplatte (3) erstreckt.

11. Schienbeinimplantat (2) nach einem der Ansprüche 1 bis 10, wobei die Schienbeingrundplatte (3) ein Außenprofil aufweist, das im Wesentlichen mit dem Außenprofil des Schienbeineinsatzes (4) identisch ist.

12. Schienbeinimplantat (2) nach einem der Ansprüche 1 bis 11, wobei der Schienbeineinsatz (4) ausgestaltet ist, um stationär auf der Schienbeingrundplatte (3) installiert zu sein.

## Claims

1. A tibial implant (2) for knee prosthesis, comprising:
- a tibial base (3) intended to be fastened to a resected proximal end of a tibia of a patient, the tibial base (3) comprising a medial bearing portion (7) and a lateral bearing portion (8), the lateral bearing portion (8) comprising a postero-lateral edge (12) and an antero-lateral edge (13),
- a tibial insert (4) shaped to be mounted on the tibial base (3), the tibial insert (4) comprising:
- a medial insert portion (17) including a concave medial bearing surface (18) intended to cooperate with a medial condyle of a femoral component or of a femur, the medial insert portion (18) comprising a postero-medial edge (22) and an antero-medial edge (23), and
- a lateral insert portion (19) including a concave lateral bearing surface (21) intended to cooperate with a lateral condyle of the femoral component or of the femur, the lateral insert portion (19) comprising a postero-lateral edge (24) and an antero-lateral edge (25),
**characterized in that** the lateral insert portion (19) has, at its postero-lateral edge (24), an insert notch (26) for the passage of the tendon of the popliteus, and/or **in that** the lateral bearing portion (8) has, at its postero-lateral edge (12), a base notch (13) for the passage of the tendon of the popliteus.

2. The tibial implant (2) according to claim 1, wherein the insert notch (26) is shaped so that, in use, the postero-lateral edge (24) of the lateral insert portion (19) extends, at said insert notch (26), recessed from the cortical contour of the resected proximal end of the tibia.

3. The tibial implant (2) according to claim 1 or 2, wherein the insert notch (26) is located in an angular sector (27) centred on the intersection axis (O) between the median antero-posterior plane (C) and the median medio-lateral plane (D) of the tibial insert (4), said angular sector (27) including a first side (28) oriented at an angle comprised between 20 and 40° relative to the median antero-posterior plane (C) of the tibial insert (4), and a second side (29) oriented at an angle comprised between 60 and 80° relative to the median antero-posterior plane (C) of the tibial insert (4).

4. The tibial implant (2) according to any one of claims 1 to 3, wherein the insert notch (26) extends over the entire height of the tibial insert (4).

5. The tibial implant (2) according to any one of claims 1 to 4, wherein the antero-lateral edge (25) of the lateral insert portion (19) and the antero-medial edge (23) of the medial insert portion (17) have profiles corresponding respectively substantially to the antero-lateral and antero-medial cortical contours of the resected proximal end of the tibia.

6. The tibial implant (2) according to any one of claims 1 to 5, wherein the postero-medial edge (22) of the medial insert portion (17) has a profile substantially corresponding to the postero-medial cortical contour of the resected proximal end of the tibia.

7. The tibial implant (2) according to any one of claims 1 to 6, wherein the postero-lateral edge (24) of the lateral insert portion (19) includes a first edge portion (24a) delimiting the insert notch (26) and at least one second edge portion (24b), the second edge portion (24b) having a profile substantially corresponding to a respective portion of the postero-lateral cortical contour of the resected proximal end of the tibia.

8. The tibial implant (2) according to any one of claims 1 to 7, wherein the base notch (13) is shaped so that, in use, the postero-lateral edge (12) of the lateral bearing portion (8) extends, at said base notch (13), recessed from the cortical contour of the resected proximal end of the tibia.

9. The tibial implant (2) according to any one of claims 1 to 8, wherein the base notch (13) is located in an angular sector (14) centred on the intersection axis (I) between the median antero-posterior plane (A) and the median medio-lateral plane (B) of the tibial base (3), said angular sector (14) including a first side (28) oriented at an angle comprised between 20 and 40° relative to the median antero-posterior plane (A) of the tibial base (3), and a second side (29) oriented at an angle comprised between 60 and 80° relative to the median antero-posterior plane (A) of the tibial base (3).

10. The tibial implant (2) according to any one of claims 1 to 9, wherein the base notch (13) extends over the entire height of the tibial base (3).

11. The tibial implant (2) according to any one of claims 1 to 10, wherein the tibial base (3) has an outer profile substantially identical to the outer profile of the tibial insert (4).

12. The tibial implant (2) according to any one of claims 1 to 11, wherein the tibial insert (4) is shaped to be fixedly mounted on the tibial base (3).
